# FASCICULE DE BREVET EUROPEEN

(11) **EP 3 096 717 B1**
(45) Date de publication et mention de la délivrance du brevet: **24.07.2019**
(21) Numéro de dépôt: 15700889.7
(22) Date de dépôt: 22.01.2015
(51) Int. Cl.: A61F 5/08, A61F 5/56, A61M 29/00

(54) **DISPOSITIF POUR FACILITER LA RESPIRATION NASALE CHEZ LES RONFLEURS**
VORRICHTUNG UM DIE ATMUNG DURCH DIE NASE BEI SCHNARCHENDEN ZU VEREINFACHEN
DEVICE FOR FACILITATING NASAL RESPIRATION FOR PEOPLE WHO SNORE

(30) Priorité: 22.01.2014 FR 1450518
(43) Date de publication de la demande: 30.11.2016
(73) Titulaire: Alglave, Michel, 97440 Saint-Andre (Ile de la Réunion) (FR)
(72) Inventeur: Alglave, Michel, 97440 Saint-Andre (Ile de la Réunion) (FR)
(74) Mandataire: Decobert, Jean-Pascal
(86) Numéro de dépôt international: PCT/EP2015/051283
(87) Numéro de publication internationale: WO 2015/110550

(56) Documents cités:
- WO-A1-2014/012827
- US-A1- 2004 177 852
- US-A1- 2010 326 448
- US-B1- 8 151 799

## Description

### DOMAINE DE L'INVENTION

L'invention concerne un dispositif de facilitation de respiration nasale pour ronfleur. Le présent dispositif est destiné à améliorer la respiration nasale chez les ronfleurs de sorte à limiter voire supprimer les ronflements. Le dispositif trouvera son application pour le traitement des ronflements principalement à participation nasale. Le dispositif est destiné à être employé bouche fermée.

Le document US 8,151,799 A1 est regardé comme l'état de la technique le plus proche.

### ETAT DE LA TECHNIQUE

Le ronflement est le bruit respiratoire que produit un dormeur. Ce bruit traduit la vibration des tissus de la gorge détendus par le sommeil. Pendant le sommeil, la respiration buccale entraîne un double mouvement d'ouverture et de recul de la mâchoire inférieure, réduisant le passage de l'air dans la gorge et provoquant la vibration du voile du palais. La respiration buccale est notamment due à une obstruction nasale. L'habitude de respirer par la bouche devient un réflexe impossible à dominer.

Il existe de nombreux dispositifs destinés à réduire les ronflements du dormeur. Ces dispositifs s'apparentent tous à des orthèses d'avancée mandibulaire. Ces dispositifs médicaux permettent de maintenir la mandibule, mâchoire inférieure, en position avancée pendant le sommeil. Cette avancée mécanique libère le passage de l'air au niveau du pharynx et diminue le phénomène d'obstruction du passage de l'air à l'arrière de la gorge. Ces dispositifs se composent d'une gouttière supérieure se positionnant sur l'arcade dentaire supérieure et une gouttière inférieure se positionnant sur l'arcade dentaire inférieure. Les deux gouttières sont reliées par des biellettes positionnées sur les faces externes des gouttières de sorte à renvoyer la mâchoire inférieure en avant.

Ces dispositifs sont assez difficiles à mettre en place correctement pour obtenir une efficacité satisfaisante, notamment les gouttières doivent être conformées par l'utilisateur à chaque utilisation. Ces dispositifs sont particulièrement gênants et les utilisateurs ont beaucoup de difficultés à les conserver dans la bouche d'autant plus si leur respiration nasale est obstruée. Ces dispositifs ne sont donc pas utiliser correctement et les résultats ne sont pas satisfaisants. De plus ces dispositifs ne traitent pas les causes nasales des ronflements.

Il existe donc le besoin de proposer un dispositif pour réduire voire limiter les ronflements qui résolve au moins une partie des inconvénients des dispositifs de l'état de l'art.

### RESUME DE L'INVENTION

A cet effet, l'invention concerne un dispositif pour faciliter la respiration nasale. Ce dispositif comprend une gouttière dotée d'une face d'appui maxillaire et de deux demi-arcs, symétriques de part et d'autre d'une direction médiane, se rejoignant en une zone avant de la gouttière, la gouttière comprend dans sa zone avant une bordure antérieure supérieure. Le dispositif est caractérisé en ce qu'il comprend un écran labial comprenant un pan supérieur agencé dans le prolongement de la bordure antérieur supérieure et s'étendant au-delà de la face d'appui maxillaire, ledit pan supérieur comprend deux extensions supérieures configurées pour former une zone d'appui du sillon gingivo-labial.

Le dispositif permet une dilatation narinaire facilitant la respiration nasale de l'utilisateur, limitant de fait la respiration buccale et donc les ronflements.

Le dispositif selon l'invention présente l'avantage d'être facile d'utilisation et de traiter une cause nasale du ronflement.

### BREVE DESCRIPTION DES FIGURES

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.
La figure 1 montre une perspective de face d'un premier mode de réalisation de l'invention.
La figure 2 montre une perspective arrière d'un premier mode de réalisation de l'invention.
La figure 3 montre une perspective de face du dispositif suivant un premier mode de réalisation de l'invention, illustrant le positionnement relatif au nez et aux narines de l'utilisateur.
La figure 4 montre une perspective suivant la coupe AA de la figure 3.
La figure 5 montre une perspective de face d'un deuxième autre mode de réalisation de l'invention avec l'écran labial amovible.
La figure 6 est une vue en perspective suivant la coupe BB- de la figure 5.
La figure 7 est une vue en coupe schématisée d'un dispositif selon l'invention positionnée dans une cavité buccale.
Les figures 8 à 12 représentent un dispositif selon l'invention en trois dimensions suivant un même mode de réalisation.

### DESCRIPTION DETAILLEE

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention porte sur un dispositif de facilitation de respiration nasale bouche fermée comprenant une gouttière dotée d'une face d'appui maxillaire et de deux demi-arcs symétriques de part et d'autre d'une direction médiane et se rejoignant en une zone avant de la gouttière, la gouttière comprend dans sa zone avant une bordure antérieure supérieure caractérise en ce qu'il comprend un écran labial comprenant un pan supérieur agencé dans le prolongement de la bordure antérieur supérieure et s'étendant au-delà de la face d'appui maxillaire, ledit pan supérieur comprenant deux extensions supérieures configurées pour former une zone d'appui du sillon gingivo-labial supérieur d'un utilisateur permettant d'augmenter le passage de l'air dans le nez.

Avantageusement, l'écran labial est convexe dans un plan sagittal et dans un plan frontal.

Avantageusement, la convexité de l'écran labial est configurée pour être apte à propulser le sillon gingivo-labial supérieur vers l'avant suivant un plan sagittal.

Avantageusement, l'écran labial comprend un pan inférieur agencé dans le prolongement de la bordure antérieure supérieure en s'étendant en deçà de la face d'appui maxillaire.

Avantageusement, les deux extensions supérieures de l'écran labial sont séparées par une incurvation configurée pour coopérer avec le sillon gingivo-nasal supérieur.

Avantageusement, le dispositif comprend un bourrelet agencé sur le pourtour du pan supérieur de l'écran labial.

Avantageusement, la gouttière comporte une face d'appui mandibulaire plane opposée à la face d'appui maxillaire.

Avantageusement, la face d'appui maxillaire et/ou la face d'appui mandibulaire comporte, au niveau d'une zone latérale distale de chaque demi-arc, une surface bombée.

Avantageusement, le dispositif comprend un rebord palatin inférieur s'étendant depuis une zone avant postérieure de la face d'appui maxillaire, ledit rebord palatin étant formé par une nappe sensiblement incurvée dont la convexité est orientée vers la gouttière.

Avantageusement, le dispositif est formé de manière symétrique suivant un plan sagittal passant par la direction médiane.

Avantageusement, au moins une partie de l'écran labial est amovible de sorte à être rapportée par des moyens de fixations rapides sur la bordure antérieure supérieure de la gouttière.

Avantageusement, le rebord palatin inférieur comprend au moins deux zones de surépaisseurs conformées de part et d'autre du plan sagittal de sorte à être en contact avec une gencive inférieure au niveau des incisives inférieures.

Avantageusement, l'écran labial présente une épaisseur aux sommets des extensions inférieure ou égale à 1cm et une hauteur s'étendant entre la face d'appui maxillaire et le sommet des extensions comprise entre 23 et 30 mm.

Avantageusement, la face d'appui mandibulaire comprend au moins une rainure longitudinale destinée à recevoir les dents inférieures par leurs crêtes.

Avantageusement, l'écran labial possède une variation de sa dureté.

Avantageusement, l'écran labial possède une décroissance de sa dureté depuis la face d'appui maxillaire en direction des deux extensions.

Avantageusement, l'écran labial possède une décroissance de sa dureté depuis l'arrière vers l'avant.

Avantageusement, les deux extensions ne s'étendent pas au-delà de la largeur du nez de l'utilisateur au niveau des narines.

Dans la description qui suit et à moins qu'il en soit indiqué autrement, on entend par symétrique le fait que le dispositif de l'invention présente avantageusement une symétrie, soit pour certaines parties suivant un axe de direction médiane qui sera explicité plus loin, soit suivant un plan du type sagittal. Le terme symétrique ne signifie pas que de légères variations dimensionnelles ou géométriques soient impossibles autour d'une symétrie au sens strict. Notamment, suivant les corrections à apporter à la morphologie de l'utilisateur, le dispositif peut présenter de légères variations entre les parties latérales gauche et droite de l'invention aptes à coopérer avec les arcades dentaires. Le terme « plan sagittal » s'entend de sa définition anatomique habituelle comme d'un plan vertical et perpendiculaire au plan vue de face. Typiquement, ce plan est parallèle à un axe antéropostérieur de la tête et passe par un point situé à mi-distance entre les deux yeux. Dans le cadre de l'invention, la direction médiane est incluse dans le plan sagittal, le plan sagittal pouvant être défini par ailleurs comme le plan contenant ladite direction médiane et sensiblement perpendiculaire à la face d'appui maxillaire qui sera présentée plus en détails ultérieurement. L'adjectif « frontal » est ici employé pour désigner une direction d'intersection entre un plan frontal et le plan sagittal, c'est-à-dire encore généralement une direction verticale.

Les termes du type sous, en dessous, en deçà s'entendent d'une position relativement à une direction verticale généralement suivant l'épaisseur de la gouttière.

On entend par secteur d'arcade dentaire, une portion anatomique de l'utilisateur pouvant comprendre une partie osseuse et/ou une partie dentaire au niveau de laquelle le dispositif peut s'appliquer.

L'adjectif « maxillaire » est utilisé en référence à la mâchoire supérieure et l'adjectif « mandibulaire » en référence à la mâchoire inférieure.

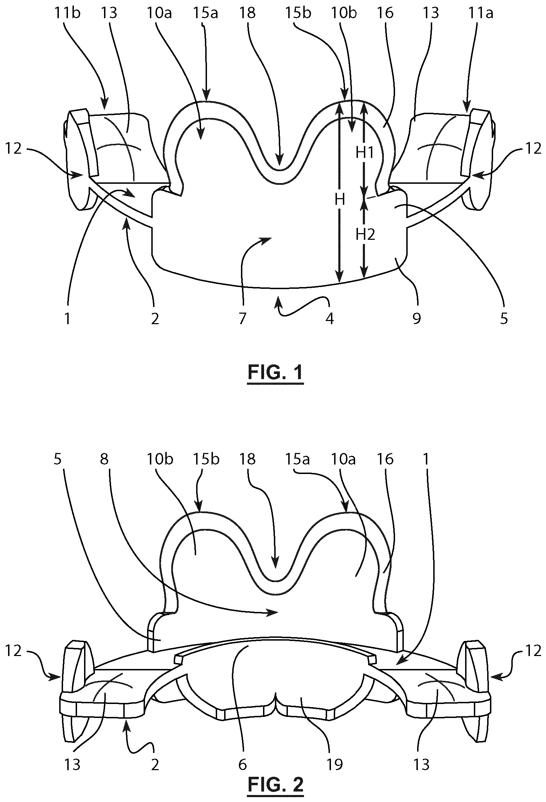

Le dispositif selon l'invention apparaît sous différentes vues aux figures 1 à 7.

Il présente globalement une forme adaptée aux contours de l'arcade dentaire supérieure de l'utilisateur destiné à recevoir le dispositif.

Comme pour des dispositifs d'avancée mandibulaire connus, le dispositif de l'invention comporte une gouttière permettant au moins un appui de la mâchoire supérieure. A cet effet, la gouttière comprend une face d'appui maxillaire 1, visible par exemple en perspective en figure 2. Avantageusement mais non exclusivement, la face d'appui maxillaire 1 est sensiblement plane hormis dans une zone latérale distale 11a, 11b qui sera explicitée plus loin. La face d'appui maxillaire 1 et sa surface bombée 13 permet l'application des dents de l'arcade dentaire supérieure de l'utilisateur.

A l'opposé de la face d'appui maxillaire 1, la gouttière comporte préférentiellement une face d'appui mandibulaire 2 qui peut être de configuration sensiblement identique à la face d'appui maxillaire 1 sauf qu'elle est préférentiellement plane alors que la face maxillaire 1 est avantageusement dotées de surfaces bombées 13 présentées en détail ultérieurement.

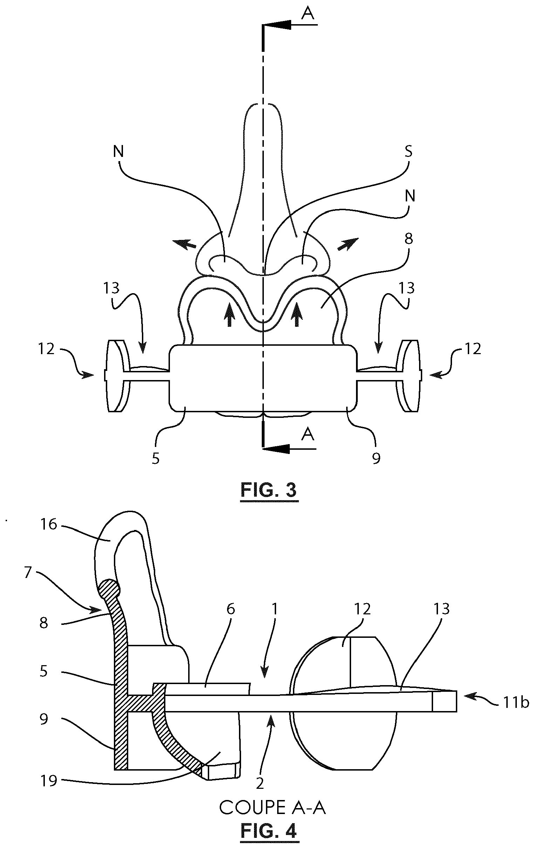

D'une manière générale, pour suivre le contour anatomique de l'arcade dentaire supérieure de l'utilisateur, la gouttière comporte deux demi-arcs 3a, 3b, chacun placé d'un côté de la gouttière autour d'une zone avant 4 réalisant la jonction entre les deux demi-arcs 3a, 3b symétriques. L'axe de symétrie des deux demi-arcs 3a, 3b correspond à une direction médiane m de la gouttière qui la coupe en deux parties égales. Cette direction « m » est visible notamment sur la vue de face, figure 3, et correspond à l'intersection entre le plan sagittal et un plan sensiblement horizontal dans lequel se situe au moins approximativement la face d'appui maxillaire 1.

On comprend aisément que la forme et la dimension de la gouttière sont adaptées à la morphologie de l'utilisateur et peuvent donc être variables. En conséquence, l'invention est ainsi déclinable en plusieurs tailles. La gouttière est avantageusement souple et donc déformable élastiquement comme indiqué précédemment et peut être réalisée dans différents matériaux biocompatibles et particulièrement du polychlorure de vinyle (PVC) ou encore du silicone.

Dans la partie présentant la face d'appui maxillaire 1 et la face d'appui mandibulaire 2, la gouttière constitue un plan de morsure s'interposant entre l'arcade du haut et celle du bas. Son épaisseur typique est de l'ordre de 2 à 4 millimètres.

La gouttière comprend une bordure antérieure supérieure 5 et avantageusement une bordure postérieure supérieure 6. Ces bordures 5 - 6 sont positionnées dans la zone avant 4 de la gouttière, au-delà de la face d'appui maxillaire 1. La bordure antérieure 5 est la bordure agencée à l'extérieure de la concavité de la gouttière tandis que la bordure postérieure 6 est la bordure agencée à l'intérieure de la concavité de la gouttière. Avantageusement les bordures supérieures antérieure 5 et postérieure 6 suivent la convexité de la gouttière. Les bordures s'étendent sensiblement dans un plan perpendiculaire au plan de la face d'appui maxillaire. Ces bordures 5-6 forment des guides antérieurs et postérieurs pour l'arcade dentaire maxillaire, notamment pour guider les dents dans la gouttière.

Selon l'invention, outre la gouttière, le dispositif comprend des moyens permettant, sur la base d'une poussée exercée par la langue de l'utilisateur et d'un serrage de la gouttière entre la mâchoire supérieure et la mâchoire inférieure, de transférer ces efforts au niveau du sillon gingivo-labial. On entend par sillon gingivo-labial, le sillon supérieur c'est à dire celui de la maxillaire. Plus précisément, le sillon gingivo-labial s'entend comme l'espace situé entre la lèvre supérieure et la gencive supérieure et comprenant au moins le frein labial supérieur.

Pour parvenir à l'action souhaitée, le dispositif comprend un écran labial 7 dont des modes de réalisation sont visibles aux figures 1 à 7.

L'écran labial 7 est situé dans la zone antérieure du dispositif de l'invention et constitue la partie frontale de l'ensemble. Sa forme est avantageusement convexe. Avantageusement, la convexité est exécutée non seulement suivant des plans parallèles au plan sagittal mais aussi suivant des plans perpendiculaires. L'écran labial 7 présente une convexité dans le plan sagittal et dans le plan frontal. Cette convexité de l'écran labial 7, plus précisément ces deux convexités permettent une avancée du sillon gingivo-labial suivant un plan sagittal. L'écran labial 7 est agencé dans le prolongement de la bordure antérieure supérieure 5. Préférentiellement, l'écran labial 7 comprend un pan supérieur 8 s'étendant depuis la bordure antérieure supérieure 5 et au-delà de la face d'appui maxillaire 1.

Selon l'invention, le pan supérieur 8 de l'écran labial 7 comprend deux extensions supérieures 10a, 10b se prolongeant jusqu'au sillon gingivo-labial supérieur en regard des narines. La forme en V, c'est-à-dire produisant un écartement progressif des extensions vers leurs extrémités distales 15a-15b, est destinée à respecter le frein labial supérieur. Lorsque l'utilisateur serre les dents, ces deux extensions 10a, 10b vont améliorer la respiration nasale de l'utilisateur. Les deux extensions 10a, 10b exercent simultanément une poussée vers l'avant dans un plan sagittal, une poussée verticale vers le haut et une poussée latérale, sensiblement en oblique en écartement dans un plan frontal. Cette combinaison de poussée entraine une modification du nez améliorant la respiration nasale. Le passage de l'air dans le nez est augmenté. Plus précisément, ces poussées modifient la forme des narines. De même, ces poussées modifient les conduits nasaux et notamment la valve de chaque conduit, améliorant la respiration nasale. On parle généralement d'une dilatation des narines. Avantageusement, le diamètre des orifices narinaires en est augmenté transversalement, permettant une meilleure ventilation nasale. Ceci est particulièrement intéressant car la majorité des ronfleurs présentent un problème de respiration nasale déficiente. Les deux extensions 10a, 10b sont séparées par une incurvation 18 dont la forme est préférentiellement configurée pour ménager un espace pour le frein labial et être atraumatique. L'incurvation forme un point bas au niveau du plan sagittal. La hauteur du pan supérieur 8 au niveau de l'incurvation 18 est moindre par rapport à la hauteur duit pan au niveau des extrémités distales 15a, 15b des deux extensions 10a, 10b.

Préférentiellement, l'écran labial 7 comprend un pan inférieur 9 s'étendant depuis la bordure antérieure supérieure 5 et en-deçà de la face d'appui maxillaire 1.

L'écran labial 7 englobe avantageusement, le pan inférieur 9, la bordure antérieure supérieure 5 et le pan supérieur 8 avec ces deux extensions 10a-10b.

A titre d'exemple non limitatif, la hauteur de la bordure antérieure supérieure 5 est comprise entre 5mm et 10 mm préférentiellement de l'ordre de 7mm à 9mm, la hauteur de la bordure postérieure supérieure 6 est comprise entre 2 et 6mm. A titre préféré, la hauteur H de l'écran labial 7 est comprise entre 30 et 40mm, préférentiellement entre 34mm et 35mm. La hauteur h1 du pan supérieur 8 est avantageusement égale à la hauteur h2 regroupant la bordure antérieure supérieure 5 et le pan inférieur 9. A titre préféré entre 15mm et 20mm préférentiellement 17mm. Selon un mode de réalisation avantageux, il est possible de prévoir que la hauteur h1 puisse atteindre 30 mm de hauteur par exemple 27mm.

L'écran labial 7 présente par exemple une longueur de 20 à 50mm. Préférentiellement les deux extensions 10a, 10b s'étendant sur une longueur de 15 à 45mm. Cette longueur des deux extensions 10a, 10b est par exemple configurée pour correspondre à la largeur du nez de l'utilisateur au niveau des narines. Préférentiellement, les deux extensions 10a, 10b ne s'étendent pas au-delà de la partie frontale du dispositif. On entend la partie frontale comme la partie du dispositif destinée à être agencée en regard du nez de l'utilisateur. Avantageusement, les deux extensions 10a, 10b s'étendent de part et d'autre du frein labial supérieur sur une longueur totale correspondant à la largeur du nez de l'utilisateur.

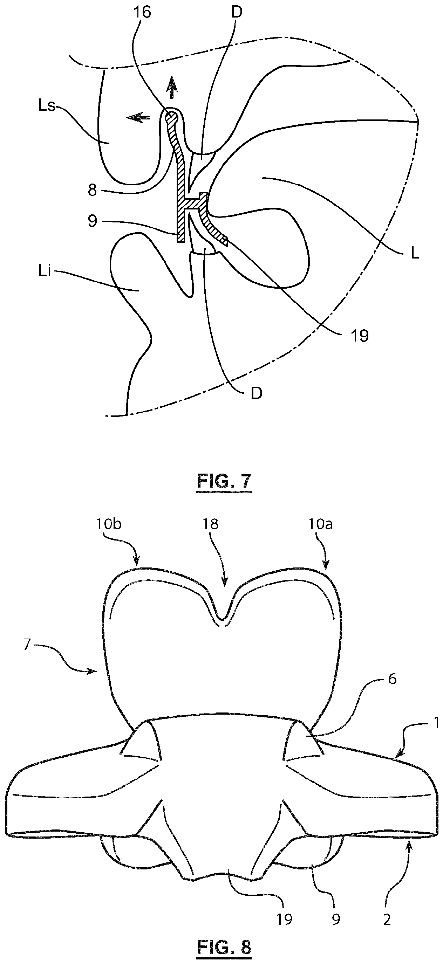

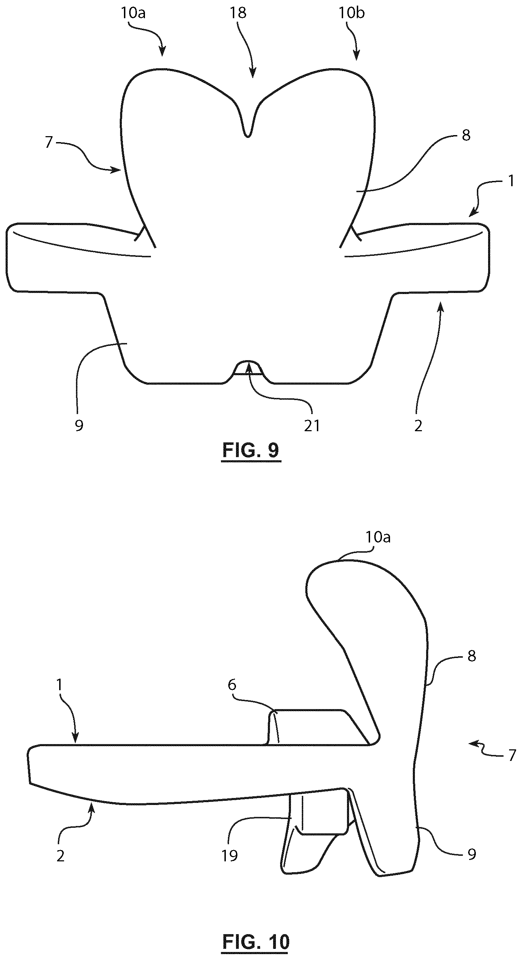

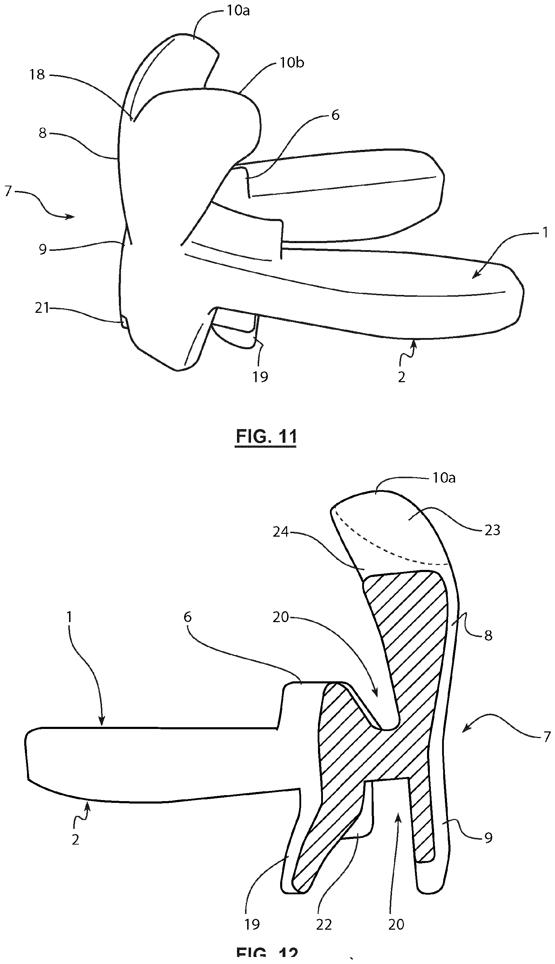

A titre d'exemple illustré aux figures 8 à 12, les extensions 10a, 10b présentent une épaisseur à leur sommet inférieure ou égale à 1cm.

L'écran labial 7 est destiné à être en contact par sa face externe avec les lèvres supérieure et inférieure de l'utilisateur. Préférentiellement, le pan supérieur 8 est destiné à être au contact de la lèvre supérieure Ls tandis que le pan inférieur 9 est destiné à être au contact de la lèvre inférieure Li.

La forme convexe de l'écran labial 7 est également destinée à être atraumatique pour l'utilisateur qui doit porter ce dispositif sur des périodes de temps longues, notamment durant la nuit entière. L'écran labial 7est configuré pour s'adapter parfaitement à la forme externe de l'arcade dentaire supérieure. A cet effet, il est préféré que l'écran labial 7 soit doté à sa périphérie préférentiellement supérieure destinée à être en contact avec le sillon gingivo-labial et plus particulièrement à la base des narines d'un bourrelet 16. Ce bourrelet 16 de section sensiblement arrondie permet d'éviter des blessures et/ou des irritations du tissu buccal. Ce bourrelet 16 présente avantageusement une plus grande souplesse que le reste du dispositif. Préférentiellement, le bourrelet 16 constitué du même matériau que le reste du dispositif. Il forme une bordure de section plus large que l'épaisseur du reste de l'écran labial 7.

Suivant une variante tendant à améliorer l'efficacité et le confort du dispositif selon l'invention, l'écran labial 7 est avantageusement conformé pour présenter une dureté variable sur sa surface. Plus spécifiquement, il est préféré que la dureté de l'écran labial 7 soit décroissante depuis la face maxillaire 1 vers les extensions 10a, 10b sur au moins une partie de son épaisseur. Ainsi, la dureté de l'écran labial 7 est de préférence plus faible au niveau des extensions 10a, 10b et plus grande au niveau de sa connexion avec la face maxillaire 1. La variation peut être progressive ou non, avec au moins deux portions de dureté différente. Cette variation suivant la hauteur de l'écran labial 7, plus précisément sur la haute h2 décrite ci-dessus assure une transmission de l'effort de propulsion généré au niveau de la face maxillaire 1 jusqu'aux extensions 10a, 10b tout assurant un déplacement vers l'avant du sommet des extensions 10a, 10b améliorant la dilatation des narines sans être traumatisant pour les tissus. La partie la plus dure transmet en effet mieux les efforts alors que la partie la moins dure est plus facilement déformable à des niveaux de contraintes réduites.

De même, il est préféré que la dureté des extensions 10a, 10b soit variable suivant direction parallèle au plan sagittal. Ainsi, la dureté des extensions 10a, 10b est plus grande en face arrière et plus faible en face avant assurant une mobilité des extensions et une transmission satisfaisante des efforts de propulsion.

A titre d'exemple, la dureté de l'écran labial 7 et plus spécifiquement des extensions 10a, 10b est de 80 shore A dans les zones de forte dureté 24 et de 60 shore A dans les zones de faible dureté 23.

Pour compléter l'efficacité du dispositif de l'invention, il comporte avantageusement une configuration particulière pour la partie au niveau de laquelle les premières molaires supérieures sont destinées à s'appliquer. Plus précisément, la face d'appui maxillaire 1 présente préférentiellement un profil bombé au niveau d'une zone d'application d'au moins une dent molaire. Telle que visible par exemple à la figure 4, cette configuration particulière est réalisée au niveau de deux zones latérales distales 11a, 11b situées sensiblement aux extrémités distales de chaque demi-arc 3a, 3b. Le profil bombé a avantageusement une hauteur maximale vers le milieu de la largeur de la face d'appui maxillaire 1. Il peut par ailleurs être bombé dans la direction transverse à la longueur de la face d'appui maxillaire 1 avec une élévation qui atteint un maximum vers le milieu de la première molaire supérieure, avant l'extrémité distale de chaque demi-arc 3a, 3b.

En complément ou de manière alternative, l'épaisseur de la gouttière peut être variable et notamment croissante en direction de l'extrémité distale de chaque demi-arc 3a, 3b au niveau des zones distales 11a, 11b. En augmentant l'épaisseur de la gouttière dans ses zones distales 11a, 11b, par exemple la zone d'appui dentaire de la face d'appui maxillaire 1 est légèrement surélevée relativement au reste de la gouttière de sorte à former la surface bombée 13. La surface bombée 13 peut également être agencée sur la face d'appui mandibulaire 2 ou bien simultanément sur les deux faces d'appui maxillaire 1 et mandibulaire 2. La surépaisseur due à la surface bombée 13 entraine un abaissement de la mandibule. Les articulations sont détendues facilitant une propulsion vers l'avant de la mandibule suivant un plan sagittal. Cette propulsion permet de mettre sensiblement à l'aplomb l'arcade dentaire maxillaire et l'arcade dentaire mandibulaire contribuant à limiter les ronflements.

Suivant une autre possibilité de l'invention les zones latérales distales 11a, 11b présentent des pans, préférentiellement des pans distaux externes 12a, 12b préférentiellement pour chaque demi-arc 3a, 3b. Avantageusement, le dispositif ne comprend pas de pans distaux internes. Ces pans 12a, 12b, sont en contact externes d'une dent molaire et particulièrement la première molaire supérieure de sorte à exercer un appui latéral sur cette dernière. Cette configuration de pans permet de fixer correctement le dispositif sans toutefois exercer d'effort important sur la dentition. Les pans 12a, 12b, sont préférentiellement pourvus d'un profil particulier suivant un plan de coupe perpendiculaire au plan sagittal présentant des renflements rentrant progressivement au-dessus de la face d'appui maxillaire 1. Les pans 12a, 12b s'étendent sensiblement perpendiculaire au plan de la face d'appui maxillaire 1 et préférentiellement au-delà et en deçà de ladite face.

Sur la face opposée à la face d'appui maxillaire 1, la face d'appui mandibulaire 2 est cependant avantageusement présente avec au moins un rebord en saillie autour de ladite face d'appui mandibulaire 2.

Préférentiellement, la face d'appui mandibulaire 2 comprend au moins une rainure longitudinale. Cette rainure longitudinale s'étend avantageusement sur la longueur de l'écran labial 7. La rainure longitudinale est conformée de sorte à recevoir les crêtes des dents inférieures, plus précisément les incisives et les canines. La rainure forme ainsi une position prédéfinie de propulsion mandibulaire aidant l'utilisateur à trouver une position de propulsion satisfaisante tout en assurant un maintien en position de propulsion. Selon une possibilité préférée, le dispositif comprend au moins deux rainures longitudinales parallèles. Les rainures définissent des positions différentes de propulsion mandibulaire permettant une progression de la propulsion. L'utilisateur va utiliser tout d'abord la rainure générant la plus faible propulsion puis progressivement il utilisera la rainure générant la forte propulsion.

Avantageusement, le dispositif comprend un rebord palatin inférieur 19 apte à former une zone de l'espace dans laquelle l'extrémité distale de la langue peut être reçue et permet de guider le mouvement des incisives inférieures lors des mouvements de la mandibule. Le rebord palatin inférieur 19 s'étendant depuis la bordure postérieure supérieure 6 de la gouttière. Le rebord palatin 19 est formé d'une nappe sensiblement incurvée dont la convexité est orientée vers la gouttière. Le rebord palatin inférieur 19 est avantageusement de la forme d'un secteur de sphère ou d'hémisphère sur une portion angulaire par exemple inférieure à un quart de sphère. Le rebord palatin inférieur 19 est principalement configuré pour guider la pointe de la langue vers l'avant et le haut suivant un plan sagittal. Le rebord palatin 19 accompagne la pointe de la langue limitant son accumulation en arrière de la bouche et limitant de ce fait les ronflements. De plus, le rebord palatin inférieur 19 forme un guide de positionnement des dents de l'arcade dentaire mandibulaire et notamment des incisives dans la gouttière au niveau de la face d'appui mandibulaire. Avantageusement, le rebord palatin inférieur 19 comprend au moins deux zones de surépaisseurs dites frontales destinées à assurer un contact avec la gencive inférieur plus précisément au niveau des incisives inférieures de sorte à limiter voire réduire le contact direct avec les dents. Les surépaisseurs permettent une approche par la face linguale de l'os mandibulaire. L'os présente couramment un torus qui s'entend comme un os de plus grande dureté que l'os alentour. Ceci permet d'éviter la vestibulation des incisives c'est-à-dire une propulsion vers l'avant des incisives. Lesdites surépaisseurs sont avantageusement disposées de manière symétrique de part et d'autres du plan sagittal. Selon une possibilité préférée, la largeur de la gouttière 20 au niveau de la face mandibulaire 2 est plus importante que la largeur de ladite gouttière 20 au niveau de la face maxillaire 1. Plus précisément, le rebord palatin inférieur 19 est en retrait par rapport à la bordure postérieure supérieure, le pan inférieur 9 est à l'aplomb du pan supérieur 8 de l'écran labial 7.

Le rebord palatin inférieur 19 s'étend préférentiellement sur un secteur angulaire plus étroit que le secteur angulaire sur lequel s'étend l'écran labial 7. Préférentiellement, le rebord palatin inférieur 19 s'étend sur un secteur angulaire de l'ordre de 30° de la gouttière.

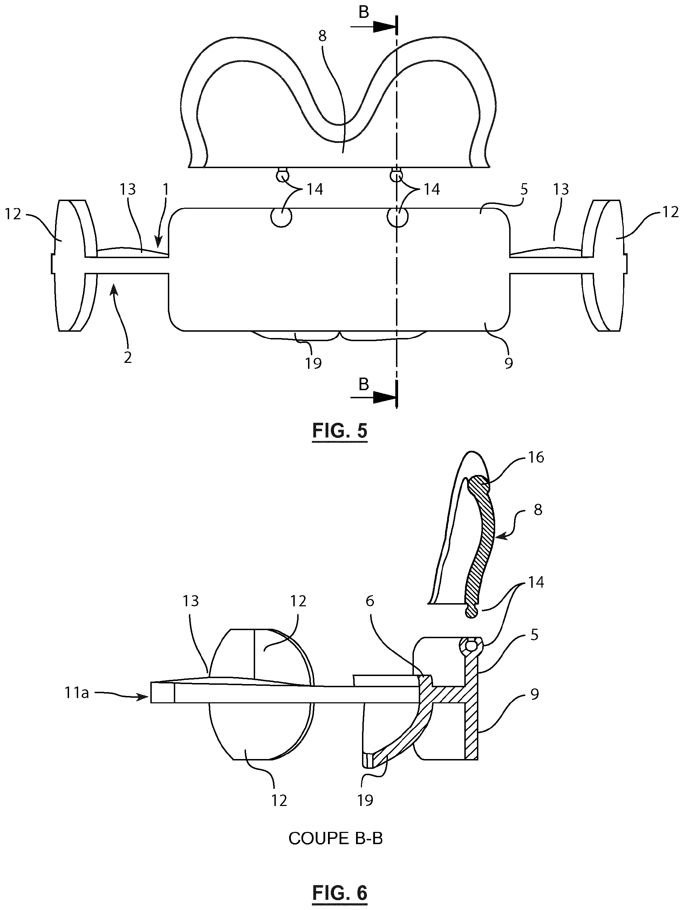

Suivant un mode de réalisation illustré aux figures 5 et 6, le pan supérieur 8 de l'écran labial 7 est au moins en partie amovible. Le dispositif comprend des moyens de fixations rapides 14 du pan supérieur 8 de l'écran labial 7 sur la gouttière et préférentiellement sur la bordure antérieur supérieure 5. Ces moyens de fixations rapides permettent de fixer un pan supérieur 8 et donc de former un écran labial 7 tel que décrit ci-dessus pour faciliter la respiration nasale sur une gouttière de base par exemple sur un appareil d'orthodontie classique. A titre d'exemple non limitatif, ces moyens de fixations rapides 14 sont par exemple des systèmes males-femelles tels que des encoches et des saillies complémentaires sur la bordure antérieure supérieure 5 et sur le pan supérieur 8. On peut ainsi former une partie de base de dispositif apte à coopérer sélectivement avec un parmi plusieurs pans supérieurs 8 en particulier pour une adaptation simple à différents anatomies d'utilisateurs.

On décrit ci-après un mode opératoire de l'invention.

La mandibule de l'utilisateur qui serre les dents se place dans le canal formé par la gouttière au niveau de la face d'appui maxillaire 1 en propulsion pour obtenir une position avantageusement de bout à bout des incisives supérieures et inférieures. Avantageusement, la gouttière rentre dans la bouche de l'utilisateur en étant contractée transversalement entre le pouce et l'index.

Au préalable, on aura pu régler les dimensions du dispositif à la morphologie de l'utilisateur et en particulier thermoformer la gouttière selon la dimension transversale souhaitée en fonction de la taille du palais de l'utilisateur.

En serrant les dents, les extrémités distales 15a, 15b des extensions 10a-10b de l'écran labial 7 vont exercer simultanément une poussée verticale en direction de la base des narines et une poussée horizontale suivant une direction parallèle à la direction médiane m orientée vers l'avant du dispositif. Cette combinaison de poussées contribue à éloigner la partie supérieure de la lèvre supérieure de l'arcade dentaire supérieure notamment en élargissant ainsi le diamètre des narines.

L'appareil agit ainsi essentiellement sur l'effet de la contracture réflexe de l'utilisateur. Néanmoins, en serrant simplement les dents dans la gouttière 1, l'utilisateur active le dispositif du simple fait de son élasticité.

On notera l'intérêt dans le dispositif d'avoir des parties en saillie au-delà des faces d'appui maxillaire 3 et mandibulaire 2 qui ne soient pas en continuité le long des demi-arcs 23a, 23b. En particulier, dans l'exemple représenté, des portions de gouttière ne présentent pas d'autre matière que l'épaisseur de la gouttière 1 entre lesdites faces 2, 3, par exemple entre les pans 16a, 16b et l'écran labial 7. De cette façon, on conserve une large possibilité de modification de déformation élastique.

### REFERENCES

- 1.: Face d'appui maxillaire
- 2.: Face d'appui mandibulaire
- 3. a - 3.b: Demi-arc
- 4.: Zone avant
- 5.: Bordure antérieure supérieure
- 6.: Bordure postérieure supérieure
- 7.: Écran labial
- 8.: Pan supérieur
- 9.: Pan inférieur
- 10.a - 10.b: Extensions supérieures
- 11.a - 11.b: Zone latérale distale
- 12.: Pan distal externe
- 13.: Surface bombée
- 14.: Moyens de fixations rapides
- 15.a - 15.b: Extrémités distales
- 16.: Bourrelet
- 18.: Incurvation
- 19.: Rebord palatin inférieur
- 20.: Gouttière
- 21.: Incurvation
- 22.: Surépaisseur
- 23.: Zone de faible dureté
- 24.: Zone de forte dureté
- m.: Direction médiane
- Ls.: Lèvre supérieure
- Li.: Lèvre inférieure
- L.: Langue
- S.: Sillon gingivo labial en V
- N.: Narine
- D.: Dent
- H.: Hauteur écran labial
- h1.: Hauteur pan supérieur
- h2.: Hauteur bordure antérieure supérieure + pan inférieur

## Revendications

1. Dispositif de facilitation de respiration nasale bouche fermée comprenant une gouttière dotée d'une face d'appui maxillaire (1) et de deux demi-arcs (3a,3b) symétriques de part et d'autre d'une direction médiane (m) et se rejoignant en une zone avant (4) de la gouttière, la gouttière comprend dans sa zone avant (4) une bordure antérieure supérieure (5), le dispositif comprenant un écran labial (7) convexe dans un plan sagittal et dans un plan frontal comprenant un pan supérieur (8) agencé dans le prolongement de la bordure antérieur supérieure (5) et s'étendant au-delà de la face d'appui maxillaire (1), ledit pan supérieur (8) comprenant deux extensions supérieures (10a,10b), ladite gouttière comportant une face d'appui mandibulaire (2) plane opposé à et en regard de la face d'appui maxillaire (1), **caractérisé en ce que** le dispositif comprend un rebord palatin inférieur (19) s'étendant depuis ladite zone avant (4) postérieure de la face d'appui maxillaire (1), ledit rebord palatin (19) étant formé par une nappe sensiblement incurvée dont la convexité est orientée vers la gouttière, et dans lequel les deux extensions supérieures (10a, 10b) s'étendent sur une longueur correspondant à la largeur du nez d'un utilisateur au niveau des narines, séparées par une incurvation (18) configurée pour coopérer avec le sillon gingivo-nasal supérieur de l'utilisateur, et
dans lequel chaque extension supérieure (10a, 10b) comprend une extrémité distale (15a, 15b) de poussée de la base d'une narine, les extensions supérieures (10a, 10b) se prolongeant depuis leurs extrémités distales (15a, 15b) l'une vers l'autre produisant une forme en V formant l'incurvation (18), les extensions supérieures (10a, 10b) étant configurées pour former une zone d'appui du sillon gingivo-labial supérieur de l'utilisateur permettant d'augmenter le passage de l'air dans le nez avec simultanément une poussée vers l'avant dans un plan sagittal, une poussée verticale vers le haut et une poussée latérale, sensiblement en oblique en écartement dans un plan frontal.

2. Dispositif selon la revendication précédente dans lequel la convexité de l'écran labial (7) est configurée pour être apte à propulser le sillon gingivo-labial supérieur vers l'avant suivant un plan sagittal.

3. Dispositif selon l'une quelconque des revendications précédentes dans lequel l'écran labial (7) comprend un pan inférieur (9) agencé dans le prolongement de la bordure antérieure supérieure (5) en s'étendant en deçà de la face d'appui maxillaire (1).

4. Dispositif selon l'une quelconque des revendications précédentes comprenant un bourrelet (16) agencé sur le pourtour du pan supérieur (8) de l'écran labial (7).

5. Dispositif selon l'une quelconque des revendications précédentes dans lequel le rebord palatin inférieur (19) comprend au moins deux zones de surépaisseurs (22) conformées de part et d'autre du plan sagittal de sorte à être en contact avec une gencive inférieure au niveau des incisives inférieures.

6. Dispositif selon l'une quelconque des revendications précédentes, formé de manière symétrique suivant un plan sagittal passant par la direction médiane (m).

7. Dispositif selon l'une quelconque des revendications précédentes dans lequel au moins une partie de l'écran labial (7) est amovible de sorte à être rapportée par des moyens de fixations rapides (14) sur la bordure antérieure supérieure (5) de la gouttière.

8. Dispositif selon l'une quelconque des revendications précédentes dans lequel l'écran labial (7) présente une épaisseur aux sommets des extensions (10a, 10b) inférieure ou égale à 1cm et une hauteur s'étendant entre la face d'appui maxillaire et le sommet des extensions (10a, 10b) comprise entre 23 et 30 mm.

9. Dispositif selon l'une quelconque des revendications précédentes dans lequel la face d'appui mandibulaire (2) comprend au moins une rainure longitudinale destinées à recevoir les dents inférieures par leurs crêtes.

10. Dispositif selon l'une quelconque des revendications précédentes dans lequel l'écran labial (7) possède une variation de sa dureté.

11. Dispositif selon la revendication précédente dans lequel l'écran labial (7) possède une décroissance de sa dureté depuis la face d'appui maxillaire (1) en direction des deux extensions (10a, 10b).

12. Dispositif selon l'une quelconque des deux revendications précédentes dans lequel l'écran labial (7) possède une décroissance de sa dureté depuis l'arrière vers l'avant.

13. Dispositif selon l'une quelconque des revendications précédentes dans lequel les deux extensions (10a, 10b) ne s'étendent pas au-delà de la largeur du nez de l'utilisateur au niveau des narines.

## Patentansprüche

1. Vorrichtung zum Vereinfachen der Atmung durch die Nase bei geschlossenem Mund, umfassend eine Rinne, ausgestattet mit einer Oberkieferunterstützungsfläche (1) und zwei symmetrischen Halbbögen (3a, 3b) auf beiden Seiten einer medialen Richtung (m), und sich in einem Vorderbereich (4) der Rinne verbindend, wobei die Rinne in ihrem Vorderbereich (4) eine vordere obere Einfassung (5) umfasst, wobei die Vorrichtung einen konvexen Labialschirm (7) in einer sagittalen Ebene und in einer frontalen Ebene umfasst, umfassend ein in der Verlängerung der vorderen oberen Einfassung (5) angeordnetes und sich ab der Oberkieferunterstützungsfläche (1) erstreckendes Oberteil (8), wobei das Oberteil (8) zwei obere Vorsprünge (10a, 10b) umfasst,
wobei die Rinne eine in der Ebene zur und hinsichtlich der Oberkieferunterstützungsfläche (1) gegenüberliegende Unterkieferunterstützungsfläche (2) umfasst, **dadurch gekennzeichnet, dass** die Vorrichtung einen unteren Gaumenrand (19) umfasst, der sich ab dem Vorderbereich (4) hinter der Oberkieferunterstützungsfläche (1) erstreckt, wobei der Gaumenrand (19) durch eine im Wesentlichen ungekrümmte Lage gebildet wird, wovon die Konvexität hin zur Rinne orientiert ist, und
wobei die zwei oberen Vorsprünge (10a, 10b) sich auf einer Länge entsprechend einer Breite einer Nase auf Höhe der Nasenlöcher erstrecken, die durch eine Krümmung (18), konfiguriert zum Kooperieren mit der oberen gingivonasalen Einkerbung des Anwenders, getrennt sind und
wobei jeder obere Vorsprung (10a, 10b) ein distales Ende (15a, 15b) der Druckausübung der Basis eines Nasenlochs umfasst, wobei die oberen Verlängerungen (10a, 10b) sich ab ihren distalen Enden (15a, 15b) hin zueinander verlängern, die Biegung (18) in V-Form bildend, wobei die oberen Vorsprünge (10a, 10b) konfiguriert sind, um einen Unterstützungsbereich der oberen gingivolabialen Einkerbung des Anwender zu bilden, die ermöglicht, in einer sagittalen Ebene den Luftdurchgang in der Nase mit einer gleichzeitigen Druckausübung nach vorne, einer vertikalen Druckausübung nach oben und einer seitlichen Druckausübung zu erhöhen, im Wesentlichen in einer vorderen Ebene schräg beabstandet.

2. Vorrichtung nach dem vorstehenden Anspruch, wobei die Konvexität des Labialschirms (7) konfiguriert ist, um in der Lage zu sein, die obere gingivolabiale Rinne der sagittalen Ebene folgend nach vorne zu bewegen.

3. Vorrichtung nach einem der vorstehenden Ansprüche, wobei der Labialschirm (7) ein in der Verlängerung des vorderen oberen Rands (5) angeordnetes, sich ab der Oberkieferunterstützungsfläche (1) erstreckendes, Unterteil (9) umfasst.

4. Vorrichtung nach einem der vorstehenden Ansprüche, umfassend einen auf dem Umfang des Oberteils (8) des Labialschirms (7) angeordneten Wulst (16).

5. Vorrichtung nach einem der vorstehenden Ansprüche, wobei der untere Gaumenrand (19) mindestens zwei Verdickungsbereiche (22) umfasst, auf beiden Seiten der sagittalen Ebene auf eine Weise gestaltet, um mit dem unteren Zahnfleisch auf Höhe der unteren Schneidezähne in Kontakt zu stehen.

6. Vorrichtung nach einem der vorstehenden Ansprüche, auf symmetrische Weise einer sagittalen Ebene folgend, die von der medialen Richtung (m) verläuft, gebildet.

7. Vorrichtung nach einem der vorstehenden Ansprüche, wobei mindestens ein Teil des Labialschirms (7) auf eine durch schnelle Fixierungsmittel (14) auf dem vorderen oberen Rand (5) der Rinne vermittelten Weise unbeweglich ist.

8. Vorrichtung nach einem der vorstehenden Ansprüche, wobei der Labialschirm (7) eine Dicke an den Scheitelpunkten der Vorsprünge (10a, 10b) aufweist, die kleiner oder gleich 1 cm ist, und eine Höhe, die sich zwischen der Oberkieferunterstützungsfläche und dem Scheitelpunkt der Vorsprünge (10a, 10b) erstreckt, zwischen 23 und 30 mm umfasst.

9. Vorrichtung nach einem der vorstehenden Ansprüche, wobei die Unterkieferunterstützungsfläche (2) mindestens eine zum Empfangen der unteren Zähne durch ihre Kuppen bestimmte längsgerichtete Aussparung umfasst.

10. Vorrichtung nach einem der vorstehenden Ansprüche, wobei der Labialschirm (7) eine Variation seiner Härte aufweist.

11. Vorrichtung nach dem vorstehenden Anspruch, wobei der Labialschirm (7) eine Abnahme seiner Härte ab der Oberkieferunterstützungsfläche (1) in Richtung der zwei Vorsprünge (10a, 10b) aufweist.

12. Vorrichtung nach einem der zwei vorstehenden Ansprüche, wobei der Labialschirm (7) eine Abnahme ihrer Härte von hinten nach vorne aufweist.

13. Vorrichtung nach einem der vorstehenden Ansprüche, wobei die zwei Vorsprünge (10a, 10b) sich nicht von der Breite der Nase des Anwenders auf Höhe der Nasenlöcher erstrecken.

## Claims

1. Device for facilitating closed-mouth nasal respiration, comprising a groove provided with a face (1) for maxillary support and two symmetrical half-arches (3a, 3b) on either side of a median direction (m) that connect to each other in a front zone (4) of the groove, the groove comprises, in its front zone (4), an upper anterior edge (5), the device comprising a labial screen (7) convex in a sagittal plane and in a frontal plane comprising an upper face (8) arranged as an extension of the upper anterior edge (5) and extending beyond the face (1) for maxillary support, said upper face (8) comprising two upper extensions (10a, 10b),
said groove comprising a flat face (2) for mandibular support opposite to and facing the face (1) for maxillary support,
**characterised in that** the device comprises a lower palatine flange (19) extending from said posterior front zone (4) of the face (1) for maxillary support, said palatine flange (19) being formed by a substantially curved plate, the convexity of which is oriented towards the groove, and
wherein the two upper extensions (10a, 10b) extend over a length corresponding to the width of the nose of a user at the nostrils, separated by a curve (18) configured to cooperate with the upper gingivo-nasal sulcus of the user, and
wherein each upper extension (10a, 10b) comprises a distal end (15a, 15b) for pushing the base of a nostril, the upper extensions (10a, 10b) extending from their distal ends (15a, 15b) towards each other producing a V shape forming the curve (18), the upper extensions (10a, 10b) being configured to form a bearing zone for the upper gingivolabial sulcus of the user that allows the passage of the air in the nose to be increased while simultaneously pushing forward in a sagittal plane, pushing upwards vertically and pushing laterally, substantially in an oblique direction and while spreading out in a frontal plane.

2. Device according to the preceding claim, wherein the convexity of the labial screen (7) is configured to be suitable for pushing the upper gingivolabial sulcus forward in a sagittal plane.

3. Device according to any one of the preceding claims, wherein the labial screen (7) comprises a lower face (9) arranged as an extension of the upper anterior edge (5) while extending below the face (1) for maxillary support.

4. Device according to any one of the preceding claims, comprising a bead (16) arranged around the contour of the upper face (8) of the labial screen (7).

5. Device according to any one of the preceding claims, wherein the lower palatine flange (19) comprises at least two zones (22) of extra thickness shaped on either side of the sagittal plane in such a way as to be in contact with a lower gum at the lower incisors.

6. Device according to any one of the preceding claims, formed symmetrically along a sagittal plane passing through the median direction (m).

7. Device according to any one of the preceding claims, wherein at least a portion of the labial screen (7) is removable in such a way as to be attached via rapid-attachment means (14) onto the upper anterior edge (5) of the groove.

8. Device according to any one of the preceding claims, wherein the labial screen (7) has a thickness at the tops of the extensions (10a, 10b) that is less than or equal to 1cm and a height extending between the face for maxillary support and the top of the extensions (10a, 10b) that is between 23 and 30mm.

9. Device according to any one of the preceding claims, wherein the face (2) for mandibular support comprises at least one longitudinal groove intended to receive the lower teeth via their crest.

10. Device according to any one of the preceding claims, wherein the labial screen (7) has a variation in its hardness.

11. Device according to the preceding claim, wherein the labial screen (7) has a decrease in its hardness from the face for maxillary support (1) towards the two extensions (10a, 10b).

12. Device according to any one of the two preceding claims, wherein the labial screen (7) has a decrease in its hardness from the rear towards the front.

13. Device according to any one of the preceding claims, wherein the two extensions (10a, 10b) do not extend beyond the width of the nose of the user at the nostrils.
